# EUROPEAN PATENT SPECIFICATION

(11) **EP 4 434 464 B1**
(45) Date of publication and mention of the grant of the patent: **07.10.2026**
(21) Application number: 24162013.7
(22) Date of filing: 07.03.2024
(51) Int. Cl.: A61B 6/00, A61B 6/04, A61B 6/50, A61B 8/08, A61B 8/00

(54) **COMPRESSION MEMBER, IMAGE GENERATION SYSTEM, AND PROGRAM**
KOMPRESSIONSELEMENT, BILDERZEUGUNGSSYSTEM UND PROGRAMM
ÉLÉMENT DE COMPRESSION, SYSTÈME DE GÉNÉRATION D'IMAGE ET PROGRAMME

(30) Priority: 20.03.2023 JP 2023043987
(43) Date of publication of application: 25.09.2024
(73) Proprietor: FUJIFILM Corporation, Tokyo 106-8620 (JP)
(72) Inventor: OSAWA, Atsushi, Kanagawa, 258-8538 (JP); HORIUCHI, Hisatsugu, Kanagawa, 258-8538 (JP); TSUBOTA, Keiji, Kanagawa, 258-8538 (JP); SAITO, Sayaka, Kanagawa, 258-8538 (JP)
(74) Representative: Dehns Germany Partnerschaft mbB

(56) References cited:
- JP-B2- 5 395 371
- US-A1- 2003 181 801
- US-A1- 2009 175 408
- US-A1- 2016 166 234
- US-A1- 2020 375 562

## Description

### BACKGROUND

### 1. Technical Field

The present disclosure relates to a compression member, an image generation system, and a non-transitory storage medium storing a program.

### 2. Related Art

JP2020-192181A discloses a radiography system for making it easier to understand a correspondence between a position on a radiation image and a position on a compression member.

The radiography system comprises a mammography apparatus that puts a breast disposed between a radiation source and the radiation detector into a compressed state, provides, at any one of a first surface irradiated with radiation by the radiation source or a second surface that is opposite to the first surface and is in contact with the breast, a compression member to which first position identification information for identifying an in-plane position of the first surface or the second surface, and captures a radiation image of the breast in the compressed state by using the radiation detector. Then, the radiography system comprises a first display controller that performs control of displaying the radiation image captured by the mammography apparatus on a display device, and a second display controller that performs control of displaying second position identification information, which indicates a position of the compression member on the radiation image corresponding to the in-plane position and corresponds to the first position identification information, on the radiation image displayed on the display device.

Incidentally, in the radiation image obtained by the radiography, both a lesion (tumor, calcification, or the like) of breast cancer and a mammary gland are displayed in white. Therefore, in a case of the breast having a high mammary gland density (so-called dense breast), the lesion of the breast cancer is hidden in a mammary gland region and is difficult to be found.

Then, there is a technique of using an ultrasound image obtained by ultrasound imaging, which is suitable for imaging the breast having the high mammary gland density as described above, in combination with a radiation image.

However, in the related art, since the ultrasound imaging is performed in a state in which the breast is not compressed and an examinee lies on a bed, it is difficult to perform registration with the radiation image of the breast obtained under the compression of the compression member in this technique.

Then, a technique of facilitating the registration between these images by acquiring both the radiation image and the ultrasound image in a state in which the breast is put into the compressed state by the compression member, and displaying both images in a state of being superimposed on each other to improve a differential determination ability of the lesion is conceivable.

However, even in this technique, the radiation image is an image obtained by an image formation technique using an electromagnetic wave, and the ultrasound image is an image obtained by an image formation technique using an elastic wave, which are images that are independently acquired, so that there is a problem that it is not easy to perform the registration accurately.

US 2009/175408 discloses a compression paddle and methods for using the same in various medical procedures.

### SUMMARY

The present disclosure has been made in view of the above circumstances, and an object thereof is to provide a compression member, an image generation system, and a non-transitory storage medium storing a program that can perform registration between a radiation image and an ultrasound image with higher accuracy than in the related art.

In order to achieve the above object, in a first aspect of the present invention there is provided a compression member as claimed in claim 1.

In an embodiment, the first material is beryllium.

In an embodiment, the first marker is disposed on the same axis as the second marker.

In an embodiment, the first marker is disposed in a patterned manner associated with position information in the compression member.

In addition, in order to achieve the above object, in a second aspect of the present invention there is provided an image generation apparatus as claimed in claim 5.

In an embodiment, the processor performs position correction of the ultrasound image and the radiation image by using a first image, which is an image of the first marker included in the ultrasound image, and a second image, which is an image of the second marker included in the radiation image, and displays the ultrasound image and the radiation image, which are subjected to the position correction, side by side or displays the ultrasound image and the radiation image, which are subjected to the position correction, in a state of being superimposed on each other.

In an embodiment, the processor performs distortion correction on at least one of the ultrasound image or the radiation image in a case in which the display is performed.

In an embodiment, the processor generates the ultrasound image by combining ultrasound images obtained in two stages of pre-scanning in which the position of the first marker provided in the compression member is used as a focal length and main-scanning in which the breast put into the compressed state by the compression member is used as a focal length.

In an embodiment, in a case in which the ultrasound image is captured, the image generation apparatus emits beams for capturing the ultrasound image from a plurality of directions toward a depth direction to combine the beams.

In addition, in order to achieve the above object, in a third aspect of the present invention there is provided a non-transitory storage medium as claimed in claim 10.

According to the present invention, it is possible to perform the registration between the radiation image and the ultrasound image with higher accuracy than in the related art.

### BRIEF DESCRIPTION OF THE DRAWINGS

Fig. 1 is a view showing an example of a schematic configuration of an image generation system according to an embodiment.
Fig. 2 is a side view showing an example of an appearance of an image generation apparatus according to the embodiment.
Fig. 3 is a three-orthographic view showing an example of a schematic configuration of a compression member according to the embodiment.
Fig. 4 is a three-orthographic view showing an example of the schematic configuration of the compression member according to the embodiment.
Fig. 5 is a bottom view showing an example of a schematic configuration of a compression part of the compression member according to the embodiment.
Fig. 6 is a view showing an example of a radiation image according to the embodiment.
Fig. 7 is a view showing an example of an ultrasound image according to the embodiment.
Fig. 8 is a bottom view showing another example of the schematic configuration of the compression part of the compression member according to the embodiment.
Fig. 9 is a view showing another example of the ultrasound image according to the embodiment.
Fig. 10 is a bottom view showing still another example of the schematic configuration of the compression part of the compression member according to the embodiment.
Fig. 11 is a view for showing a disposition surface of a first marker according to the embodiment, in which upper figures are waveform diagrams showing a reflection state of ultrasound waves in a case in which the first marker is provided on an upper surface of the compression part and a side view of the compression part, and lower figures are waveform diagrams showing a reflection state of ultrasound waves in a case in which the first marker is provided on a bottom surface (contact surface) of the compression part and a side view of the compression part.
Fig. 12 is a block diagram showing an example of a hardware configuration of a console according to the embodiment.
Fig. 13 is a block diagram showing an example of a functional configuration of the console according to the embodiment.
Fig. 14 is a flowchart showing an example of an image display process according to the embodiment.
Fig. 15 shows an upper figure which is a waveform diagram showing an example of a drive pulse for an ultrasound transducer of an ultrasound probe in a case in which the ultrasound image is generated in one scanning, and a lower figure which is a waveform diagram showing an example of a drive pulse for the ultrasound transducer of the ultrasound probe in a case in which the ultrasound image is generated by including pre-scanning.
Fig. 16 shows a left figure which is a side view showing states of the compression part and an imaging table in a case in which a breast is relatively thick, and a right figure which is a side view showing states of the compression part and the imaging table in a case where the breast is thinner than the breast shown in the left figure.
Fig. 17 is a view showing an example of a superimposition image according to the embodiment.

### DESCRIPTION OF EMBODIMENTS

Hereinafter, a description of an embodiment of the present disclosure will be made with reference to the accompanying drawings.

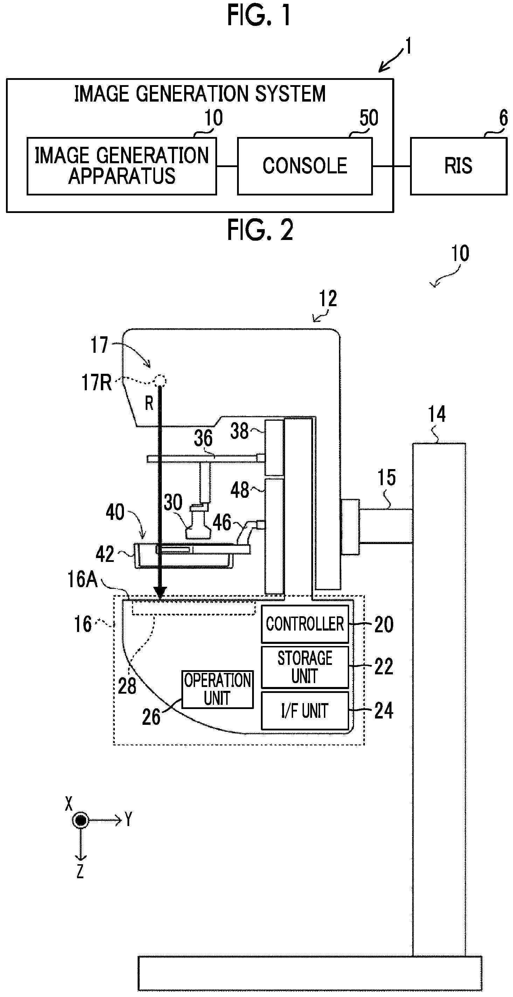

First, a description of a configuration of an image generation system 1 to which the technology of the present disclosure is applied will be made with reference to Fig. 1. Fig. 1 is a view showing an example of a schematic configuration of the image generation system 1 according to the present embodiment.

As shown in Fig. 1, the image generation system 1 comprises an image generation apparatus 10 and a console 50. The image generation apparatus 10 and the console 50, and the console 50 and an external radiology information system (RIS) 6 are configured to be connected to each other via a wired or wireless network.

In the image generation system 1 according to the present embodiment, the console 50 acquires an imaging order or the like from the RIS 6, and controls the image generation apparatus 10 in accordance with the imaging order, an instruction from the user, and the like. The image generation apparatus 10 captures a radiation image by irradiating a breast put into a compressed state between an imaging table 16 and a compression member 40, which will be described below, with radiation R. In addition, the image generation apparatus 10 acquires an ultrasound image of the breast put into the compressed state via the compression member 40.

Next, a description of a schematic configuration of the image generation apparatus 10 according to the present embodiment will be made with reference to Fig. 2. Fig. 2 is a side view showing an example of an appearance of the image generation apparatus 10 according to the present embodiment, and is a view in a case in which the image generation apparatus 10 is viewed from a right side of an examinee. As shown in Fig. 2, the image generation apparatus 10 comprises a radiation source 17R, a radiation detector 28, an imaging table 16 disposed between the radiation source 17R and the radiation detector 28, the compression member 40 that compresses the breast between the compression member 40 and the imaging table 16, and an ultrasound probe 30. In the image generation apparatus 10, a user, such as a doctor or a technician, positions the breast of the examinee on an imaging surface 16A of the imaging table 16.

The image generation apparatus 10 comprises an arm part 12, a base 14, and a shaft part 15. The arm part 12 is held to be movable in an up-down direction (Z direction) by the base 14. The shaft part 15 connects the arm part 12 to the base 14. The arm part 12 is relatively rotatable with respect to the base 14 with the shaft part 15 as a rotation axis. In addition, the arm part 12 may be relatively rotatable with respect to the base 14 with the shaft part 15 as the rotation axis separately between an upper part comprising a radiation emitting unit 17 and a lower part comprising the imaging table 16.

The arm part 12 comprises the radiation emitting unit 17 and the imaging table 16. The radiation emitting unit 17 comprises the radiation source 17R, and is configured to change an irradiation field of radiation (for example, X-rays) emitted from the radiation source 17R. For example, the change of the irradiation field may be performed by the user operating an operation unit 26, or may be performed by a controller 20 in accordance with a type of the attached compression member 40.

The imaging table 16 comprises the controller 20, a storage unit 22, an interface (I/F) unit 24, the operation unit 26, and the radiation detector 28. The controller 20 controls an overall operation of the image generation apparatus 10 in accordance with the control of the console 50. The controller 20 comprises a central processing unit (CPU), a read only memory (ROM), a random access memory (RAM), and the like (none shown). The ROM stores in advance various programs including a program executed by the CPU for performing the control related to the generation of the radiation image and the ultrasound image. The RAM transitorily stores various data.

Data of the radiation image and the ultrasound image, various types of other information, and the like are stored in the storage unit 22. The storage unit 22 is realized by, for example, a storage medium, such as a hard disk drive (HDD), a solid state drive (SSD), and a flash memory.

The I/F unit 24 performs communication of various types of information with the console 50 by wired or wireless communication. Specifically, the I/F unit 24 receives information related to the control of the image generation apparatus 10 from the console 50. Further, the I/F unit 24 transmits the data of the radiation image and the ultrasound image to the console 50.

The operation unit 26 is a part that is provided on the imaging table 16 or the like and can be operated by the user with a hand, a foot, or the like, and is, for example, a switch, a button, or a touch panel.

The radiation detector 28 is disposed in the imaging table 16, detects the radiation R transmitted through the breast and the imaging table 16, generates the radiation image based on the detected radiation R, and outputs image data indicating the generated radiation image. It should be noted that a type of the radiation detector 28 is not particularly limited and may be, for example, an indirect conversion type radiation detector that converts the radiation R into light and converts the converted light into a charge, or a direct conversion type radiation detector that directly converts the radiation R into a charge.

A probe unit 38 and a compression unit 48 are connected to the arm part 12. A support part 36 that attachably and detachably supports the ultrasound probe 30 is attached to the probe unit 38. The support part 36 (ultrasound probe 30) is moved in the up-down direction and a horizontal direction (X direction, Y direction, and Z direction) by a driving unit (not shown) provided in the probe unit 38. It is preferable that the support part 36 is formed of a material that transmits the radiation R.

The ultrasound probe 30 is used to obtain the ultrasound image of the breast put into the compressed state by the compression member 40, is disposed between the radiation source 17R and the compression member 40, irradiates the breast with ultrasound via the compression member 40, and receives the reflected waves from the breast. The probe unit 38 includes a converter (not shown) that converts the reflected waves from the breast received by the ultrasound probe 30 into the ultrasound image, and the ultrasound image is obtained by the converter.

Specifically, the ultrasound probe 30 comprises an ultrasound transducer array. The ultrasound transducer array is configured such that a plurality of ultrasound transducers are arranged one-dimensionally or two-dimensionally. The ultrasound transducer may be formed, for example, such that electrodes are formed on both ends of a piezoelectric body, such as a piezoelectric ceramic represented by lead zirconate titanate (PZT), a piezoelectric single crystal represented by lead magnesium niobate-lead titanate (PMN-PT), or a polymer piezoelectric element represented by polyvinylidene difluoride (PVDF). Also, for example, the ultrasound transducer may be a capacitive micro-machined ultrasound transducer (CMUT).

In addition, a plurality of types of the ultrasound probes 30 different from each other may be interchangeably attached to the image generation apparatus 10. Specifically, depending on a physique of the examinee (for example, a size of the breast), a tissue composition of the breast (for example, a fat mass and a mammary gland mass), a type of imaging (for example, magnification imaging and spot imaging), and the like, the ultrasound probes 30 having different performances and dimensions from each other may be attached. For example, a linear probe having a center frequency of about 7.5 MHz (for superficial use or the like), a convex probe having a center frequency of about 3.5 MHz (for abdomen or the like), and a sector probe having a center frequency of about 2.5 MHz (for heart or the like) may be used.

A support part 46 that supports the compression member 40 is attachably and detachably attached to the compression unit 48. The support part 46 (compression member 40) is moved in the up-down direction (Z direction) by a driving unit (not shown) provided in the compression unit 48.

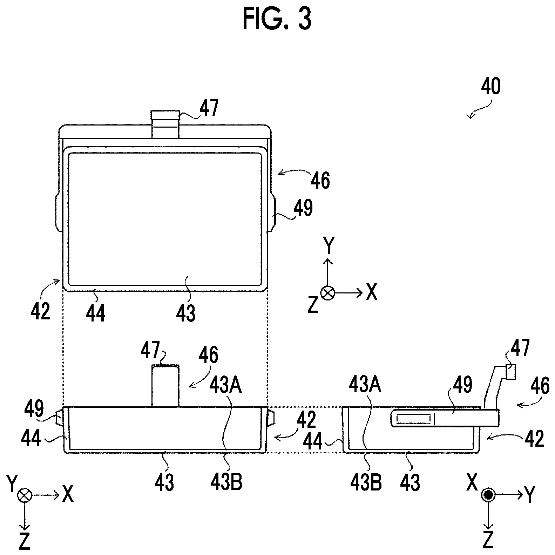

The compression member 40 is disposed between the radiation source 17R and the imaging table 16 and interposes the breast between the compression member 40 and the imaging table 16 to put the breast into the compressed state. Fig. 3 shows a three-orthographic view of an example of the compression member 40. The three-orthographic view of Fig. 3 includes a top view of the compression member 40 as viewed from above (radiation emitting unit 17 side), a side view thereof as viewed from the examinee side, and a side view thereof as viewed from the right side of the examinee. As shown in Fig. 3, the compression member 40 includes a compression part 42 and the support part 46.

The support part 46 includes an attachment part 47 and an arm 49. The attachment part 47 attaches the compression member 40 to the image generation apparatus 10, specifically, the driving unit of the compression unit 48. The arm 49 supports the compression part 42.

The compression part 42 includes a bottom part 43 formed to be substantially flat and surrounded by a wall part 44 having a substantially uniform height, and has a cross section shape formed in a recess shape. It is preferable that the compression part 42 is formed of an optically transparent or translucent material in order to perform positioning and check of the compressed state in the compression of the breast. In addition, it is preferable that the compression part 42 is formed of a material excellent in a transmittance of the radiation R and the ultrasound. In addition, it is preferable that the compression part 42 is formed of, for example, a material excellent in strength, such as drop strength and compression strength.

As such a material, for example, resin, such as polymethylpentene (PMP), polycarbonate (PC), acryl, polypropylene (PP), and polyethylene terephthalate (PET), can be used. In particular, in the polymethylpentene, an acoustic impedance, which affects the transmittance and the reflectivity of the ultrasound, is closer to an acoustic impedance of a human body (breast) than other materials, and a proportion of the noise on the ultrasound image can be decreased. Therefore, as the material of the compression part 42, the polymethylpentene is suitable.

In addition, a plurality of types of the compression members 40 different from each other may be interchangeably attached to the image generation apparatus 10. Specifically, depending on a physique of the examinee (for example, a size of the breast), a tissue composition of the breast (for example, a fat mass and a mammary gland mass), a type of imaging (for example, magnification imaging and spot imaging), and the like, the compression members 40 having different materials, sizes, and shapes from each other may be attached. For example, a compression member in accordance with the size of the breast, a compression member for axillary imaging, a compression member for magnification imaging, and a compression member for so-called spot imaging that captures the radiation image of only a region in which a lesion exists, and the like may be used. That is, the compression member 40 is not limited to the compression member that compresses the entire breast, and may have a smaller size than the breast to compress a part of the breast.

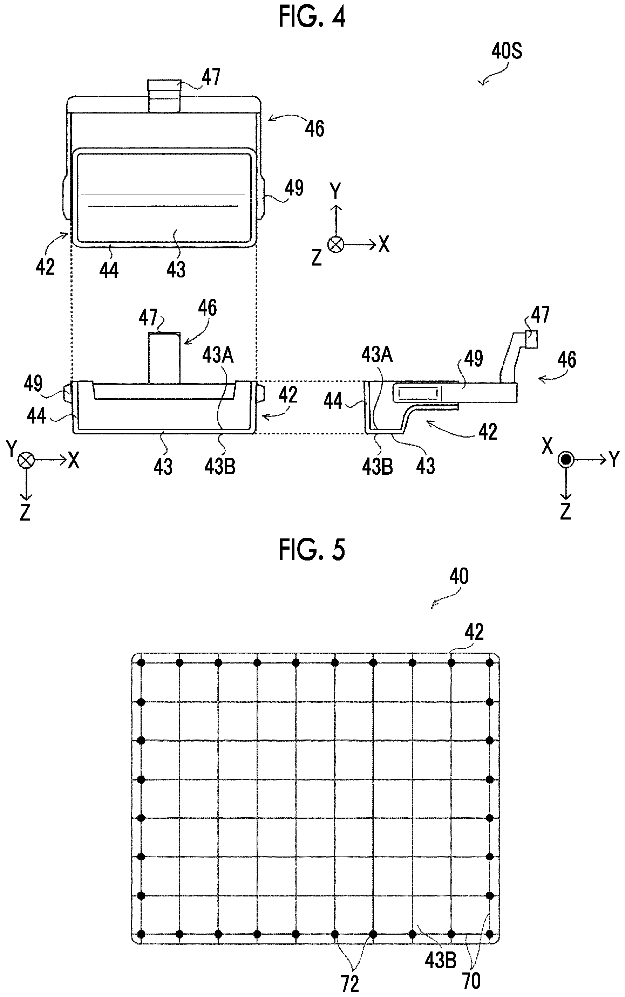

Fig. 4 shows a three-orthographic view of a compression member 40S for a small breast as an example of another form different from the compression member 40 of Fig. 3. The three-orthographic view of Fig. 4 includes a top view of the compression member 40S as viewed from above (radiation emitting unit 17 side), a side view thereof as viewed from the examinee side, and a side view thereof as viewed from the right side of the examinee. The compression member 40S includes the compression part 42 and the support part 46, as in the compression member 40 in Fig. 3. In the compression member 40S, the bottom part 43 is not flat, and the attachment part 47 side is higher than a chest wall side (side away from the attachment part 47). In addition, the height of the wall part 44 is not uniform, and a height of a part of the chest wall side is lower than a height of other parts. Due to such a shape, the compression member 40S can easily perform the positioning and the compression even for the small breast.

As described above, in the image generation apparatus 10, at least one of the compression member 40 for putting the breast into the compressed state or the ultrasound probe 30 for acquiring the ultrasound image can be attached and detached. In this case, the image generation apparatus 10 may detect the types of the compression member 40 and the ultrasound probe 30 that are attached.

For example, the attachment part 47 of the compression member 40 may be provided with a plurality of pins having different dispositions for each type of the compression member 40 as identification information, and the identification information may be read by a sensor (for example, a photointerrupter) that can detect the disposition of the pins provided in the compression unit 48. In addition, for example, a marker (for example, a bar code and a two-dimensional code) in accordance with the type of the compression member 40 may be provided at any position of the compression member 40 as identification information, and the identification information may be read by a sensor (for example, a charge coupled device (CCD) sensor) that can detect the marker.

In addition, for example, a radio frequency identification (RFID) tag having identification information in accordance with the type of the compression member 40 may be provided at any position of the compression member 40, and the identification information may be read by an RFID reader that can read the RFID tag. In addition, for example, a weight of each type of the compression member 40 and identification information may be stored in the storage unit 22 in advance in association with each other, the weight of the attached compression member 40 may be measured by a sensor that can detect the weight, and the identification information (type of the compression member 40) may be specified based on a measured value.

Similarly, for the ultrasound probe 30, the type of the attached ultrasound probe 30 may be identified in accordance with, for example, the pin, the marker, the RFID tag, or the weight.

The image generation apparatus 10 may detect a compression force applied to the breast by the compression member 40. For example, in a case in which the compression member 40 compresses the breast, a reaction force equal to the compression force is applied to the driving unit of the compression member 40. Utilizing this phenomenon, a strain gauge (for example, a load cell or the like) for detecting the reaction force applied to the driving unit may be provided in the compression unit 48 to detect and the reaction force detected by the strain gauge, as the compression force. For example, the compression force may be detected by using a semiconductor pressure sensor, a capacitive pressure sensor, and the like. In addition, for example, various sensors that detect the compression force may be provided on the compression member 40 side instead of the compression unit 48 side.

It should be noted that a gel-like or liquid medium having an ultrasound transmittance may be applied to an upper surface 43A of the bottom part 43 of the compression member 40 and/or a contact surface 43B with the breast. As such a medium, for example, a known jelly for an ultrasound examination, which has the acoustic impedance close to the acoustic impedance of the human body (breast), can be applied. That is, the image generation apparatus 10 may acquire the ultrasound image of the breast put into the compressed state by the compression member 40 in a state of being coated with the gel-like or liquid medium having the ultrasound transmittance, via the compression member 40. In this case, it is possible to suppress entry of air into an interface between an ultrasound radiation surface of the ultrasound probe 30 and the upper surface 43A and/or an interface between the contact surface 43B and the breast, and it is possible to reduce a difference in the acoustic impedance at each interface, so that the proportion of the noise applied to the ultrasound image can be decreased.

In addition, the method of imaging the breast via the image generation apparatus 10 is not particularly limited. For example, cranio-caudal (CC) imaging, medio-lateral oblique (MLO) imaging, the magnification imaging and the spot imaging for imaging a part of the breast, and the like may be performed. The CC imaging is a method of imaging the breast in the compressed state by interposing the breast between the imaging table 16 and the compression member 40 in the up-down direction (Z direction). The MLO imaging is a method of imaging the breast in the compressed state including an axilla portion by interposing the breast between the imaging table 16 and the compression member 40 in a tilted state in which a rotation angle of the arm part 12 with respect to the base 14 is equal to or greater than 45 degrees and smaller than 90 degrees.

In addition, for example, the image generation apparatus 10 may perform tomosynthesis imaging. In the tomosynthesis imaging, the radiation R is emitted from each of a plurality of irradiation positions having different irradiation angles toward the breast by the radiation source 17R, to capture a plurality of radiation images of the breast. That is, in the tomosynthesis imaging, the imaging is performed by changing the rotation angle of the radiation emitting unit 17 with respect to the base 14 while fixing the angles of the imaging table 16, the compression member 40, the breast, and the like.

In addition, in the image generation apparatus 10, the breast of the examinee may be positioned not only in a state in which the examinee is standing (standing state) but also in a state in which the examinee is sitting on a chair, a wheelchair, or the like (sitting state).

The console 50 sets an upper limit value of the compression force applied to the breast by the compression member 40 in accordance with the type of the compression member 40 attached to the image generation apparatus 10. In addition, the console 50 controls the image generation apparatus 10 to acquire the radiation image in accordance with the imaging order acquired from the RIS 6, the instruction from the user, and the like. In addition, the console 50 controls a position of the ultrasound probe 30 so that the ultrasound image can be acquired in accordance with a position of a region of interest included in the radiation image captured in the image generation apparatus 10.

As described above, the image generation apparatus 10 according to the present embodiment is provided with the probe unit 38 between the radiation emitting unit 17 and the compression unit 48 as described above, and can acquire both the radiation image and the ultrasound image while the breast is put into the compressed state by the compression member 40. Accordingly, a differential determination ability of a lesion can be improved by facilitating the registration between these images and displaying both images in a state of being superimposed on each other.

In order to enable the registration between the images of the radiation image and the ultrasound image in this case with high accuracy, two types of markers including a first marker and a second marker are provided on the contact surface 43B of the compression part 42 of the compression member 40 according to the present embodiment.

Next, a description of the first marker and the second marker according to the present embodiment will be made with reference to Fig. 5. Fig. 5 is a bottom view showing an example of a schematic configuration of the compression part 42 of the compression member 40 according to the present embodiment.

As shown in Fig. 5, the contact surface 43B of the compression part 42 of the compression member 40 according to the present embodiment is provided with a first marker 70. Since the first marker 70 is formed of a first material that is visible in the ultrasound image and is not visible in the radiation image, and is visible in the ultrasound image, a position in the compression member 40 is discriminative.

In the present embodiment, beryllium is applied as the first material. In Table 1, examples of the materials of various materials, the X-ray absorption coefficient, the acoustic velocity, the density, and the acoustic impedance are shown.

**Table 1**

| Material | Representative X-ray absorption coefficient at X-ray wavelength for medical use [µ/mm] | Acoustic velocity [m/s] | Density [kg/m³] | Acoustic impedance [Mrayl] |
|---|---|---|---|---|
| (Human body) | - | 1,460 | To 1,000 | 1.46 |
| Beryllium | To -0.045 | 12,880 | 2,340 | 30.1 |
| Graphite | To 0.1 | 18,000 | 2,200 | 39.6 |
| Aluminum | To 1.7 | 6,380 | 2,700 | 17.2 |
| Polycarbonate (compression part) | To 1.2 | 2,175 | To 1,200 | 2.61 |

As shown in Table 1, beryllium has a significantly smaller X-ray absorption coefficient than other materials, and thus beryllium is not visible in the radiation image. In addition, since beryllium has a significantly greater acoustic impedance and a higher acoustic velocity than a human body or a material of the compression part 42 (polycarbonate in the example shown in Table 1), beryllium is visible in the ultrasound image in a state in which the influence of the image of the breast or the compression part 42 is minimized. It should be noted that the first material is not limited to beryllium, and it is preferable to apply a material having a relatively small atomic number, such as graphite or aluminum. In addition to these materials, as the first material, a material that has an X-ray absorption coefficient equal to or smaller than 1.5 µ/mm and has an acoustic impedance greater than the acoustic impedance of the compression part 42 can be applied.

As shown in Fig. 5, the compression member 40 according to the present embodiment includes the first markers 70 are provided on the contact surface 43B in a grid (lattice). This is because the first marker 70 is used for distortion correction of the image, which will be described in detail below. It should be noted that, in the present embodiment, the first markers 70 form a square in a matrix. However, the present disclosure is not limited to this, and for example, the first markers 70 may have a rectangular shape in a matrix.

On the other hand, the contact surface 43B according to the present embodiment is provided with a second marker 72. The second marker 72 is provided at a position that does not affect the image interpretation of the radiation image, and is formed of a second material that is visible in the radiation image. In the present embodiment, lead is applied as the second material, but the present disclosure is not limited to this. For example, a form may be adopted in which another metal, such as iron, tungsten, or stainless steel, is applied as the second material.

As shown in Fig. 5, the compression member 40 according to the present embodiment includes the second marker 72 provided at the intersection of the grids of the first marker 70 and at the outermost intersection on the contact surface 43B. Each of the second markers 72 according to the present embodiment has a circular shape in a front view, and this shape makes it easy to distinguish the second marker 72 from a lesion, an artifact, or the like imaged in the radiation image.

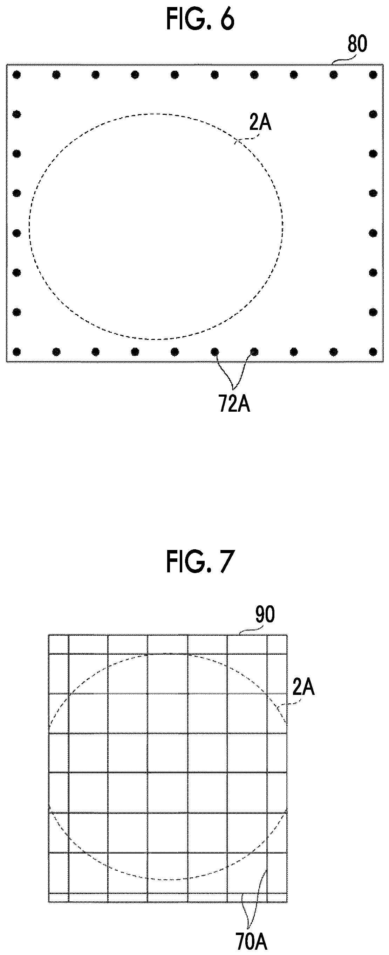

In a radiation image 80 obtained in a state in which the breast is put into the compressed state by the compression member 40 having the above configuration, as shown in Fig. 6 as an example, an image (hereinafter, referred to as a "second image") 72A of the second marker 72 is imaged at the position that does not affect the image interpretation of a breast image 2A that is the image of the breast. It should be noted that, in Fig. 6, for the sake of convenience, the second image 72A is indicated by a black circle, but in the actual radiation image, a region excluding the second image 72A and the breast image 2A is black, and the second image 72A is white.

On the other hand, in a C-plane ultrasound image 90 obtained in a state in which the compressed state of the breast by the compression member 40 is the same as the compressed state in a case of generating the radiation image 80, as shown in Fig. 7 as an example, an image (hereinafter, referred to as a "first image") 70A of the first marker 70 is imaged in a grid. It should be noted that, also in Fig. 7, for the sake of convenience, the first image 70A is indicated by a black line, but in the actual ultrasound image, a region excluding the first image 70A and the breast image 2A is black, and the first image 70A is a linear gray image with a density in accordance with the type of the first material.

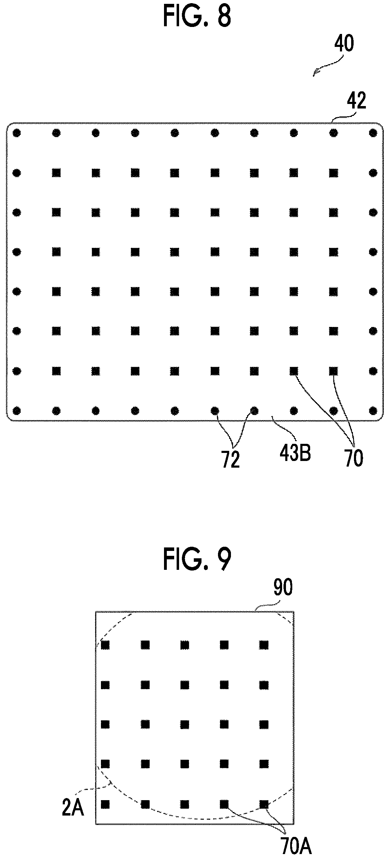

The states of the first marker 70 and the second marker 72 provided in the compression member 40 according to the present embodiment are not limited to the states shown in Fig. 5. For example, instead of providing the first marker 70 in a grid, as shown in Fig. 8 as an example, a form may be adopted in which the first marker 70 is provided in a dotted shape at a position of an intersection of the grids shown in Fig. 5 and other than the intersection at which the second marker 72 is provided. Each of the first markers 70 according to this form has a rectangular shape in a front view, and this shape makes it easy to distinguish the first marker 70 from a lesion, an artifact, or the like imaged in the image of the first marker 70 or the radiation image.

A radiation image 80 obtained in a state in which the breast is put into the compressed state by the compression member 40 having this configuration is the same as the radiation image shown in Fig. 6. On the other hand, in a C-plane ultrasound image 90 obtained in a state in which the compressed state of the breast by the compression member 40 is the same as the compressed state in a case of generating the radiation image 80, as shown in Fig. 9 as an example, the first image 70A is imaged at the intersection of the grids and the intersection other than the intersection at which the second marker 72 is provided. It should be noted that, also in Fig. 9, for the sake of convenience, the first image 70A is indicated by a black circle, but in the actual ultrasound image, a region excluding the first image 70A and the breast image 2A is black, and the first image 70A is a dot-shaped gray image with a density in accordance with the type of the first material.

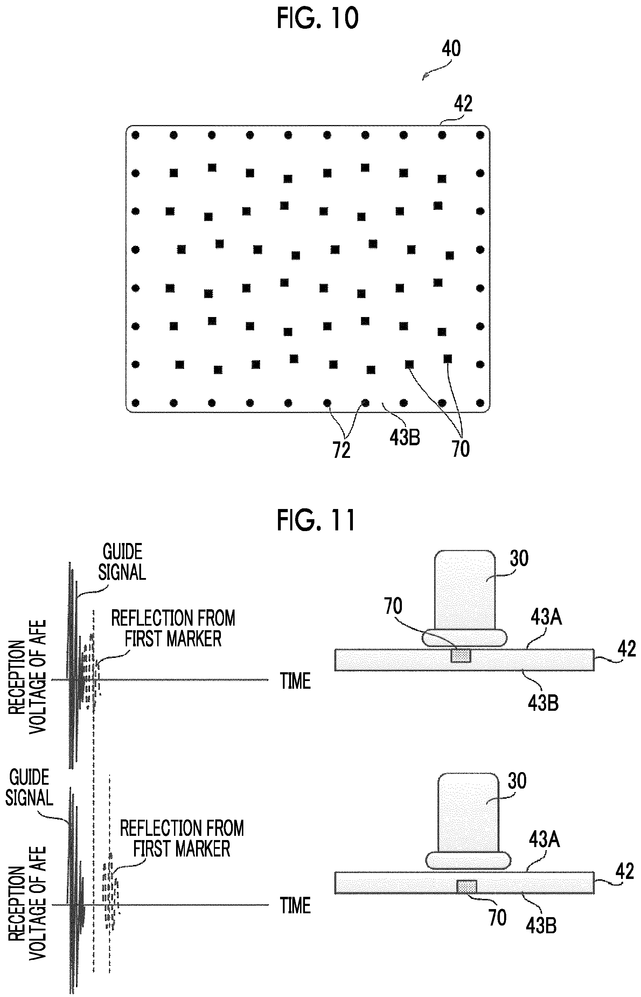

In this way, in the compression member 40 according to the present embodiment, the first marker 70 and the second marker 72 are disposed on the same axis, but the present disclosure is not limited to this. For example, as shown in Fig. 10 as an example, a form may be adopted in which the first marker 70 and the second marker 72 are not disposed on the same axis line.

In the example shown in Fig. 10, a form example is described in which the first marker 70 is disposed in a patterned manner associated with position information indicating the position in the compression part 42. It should be noted that, in the example shown in Fig. 10, a case is described in which the first marker 70 is provided in an ANOTO (ANOTO (registered trademark)) pattern.

This pattern is developed to recognize unique identification of paper or a position in a paper surface, but the position of each first marker 70 in the compression member 40 can be understood in a case in which the position information indicating the position of each dot in the compression part 42, that is, the position of the first marker 70 is registered in advance. It should be noted that the pattern is not limited to the ANOTO pattern, and any pattern can be applied as long as it is a pattern associated with the position information in the compression part 42.

In addition, in the example shown in Fig. 8, a case is described in which the shape of the first marker 70 is the rectangular shape and the shape of the second marker 72 is the circular shape, but the present disclosure is not limited to this. Any shape such as an ellipse shape, a triangle shape, a polygon shape of a pentagon or more, a star shape, a cross shape, or the like can be applied to the shape of each marker. In a case in which the shapes of the first marker 70 and the second marker 72 are set to shapes other than the circular shape or the ellipse shape, it is possible to more easily distinguish the first marker 70 and the second marker 72 from a lesion, an artifact, or the like. In addition, in a case in which the shapes of the first marker 70 and the second marker 72 are set to shapes other than the circular shape, it is possible to easily specify the distortion, the inclination, or the like of the generated ultrasound image and radiation image.

As described above, in the compression member 40 according to the present embodiment, the first marker 70 is provided on the contact surface 43B which is a surface opposite to a surface with which the ultrasound probe 30 is in contact.

For example, as shown in the upper right figure of Fig. 11 as an example, the first marker 70 can be provided on the upper surface 43A. However, in this case, it is difficult to visually recognize the first marker 70 in the ultrasound image due to multiple reflection of the ultrasound on the upper surface 43A, as shown in the upper left figure of Fig. 11.

In order to avoid this problem, in the compression member 40 according to the present embodiment, the first marker 70 is provided on the contact surface 43B as shown in the lower right figure of Fig. 11 as an example, whereby the timing of the multiple reflection is shifted and a decrease in the visibility due to the multiple reflection is suppressed as shown in the lower left figure of Fig. 11 as an example.

It should be noted that, as described above, polymethylpentene is suitable as the material of the compression part 42, and in a case in which polymethylpentene is used, a form may be adopted in which any one of the following two forms is applied, instead of the form in which a separate material, such as beryllium, is applied as the material of the first marker 70.

A first form is a form in which a material having a relatively small atomic number, such as carbon fiber, glass fiber, magnesium fiber, or cellulose nanofiber, is added to a portion of the compression part 42 provided with the first marker 70. In this form, with the addition of these additives, the portion is solid, or at least the density is changed, and thus the portion can be distinguished from the base portion as visibility in the ultrasound image.

A second form is a form in which air bubbles are put in a portion of the compression part 42 provided with the first marker 70 by a technique, such as MuCell (micro injection foam molding) (registered trademark). In this form, since the resin having a lower density than the base portion is used at a position at which the air bubbles are put, the resin can be distinguished from the base portion as visibility in the ultrasound image.

Next, a description of the console 50 according to the present embodiment will be made.

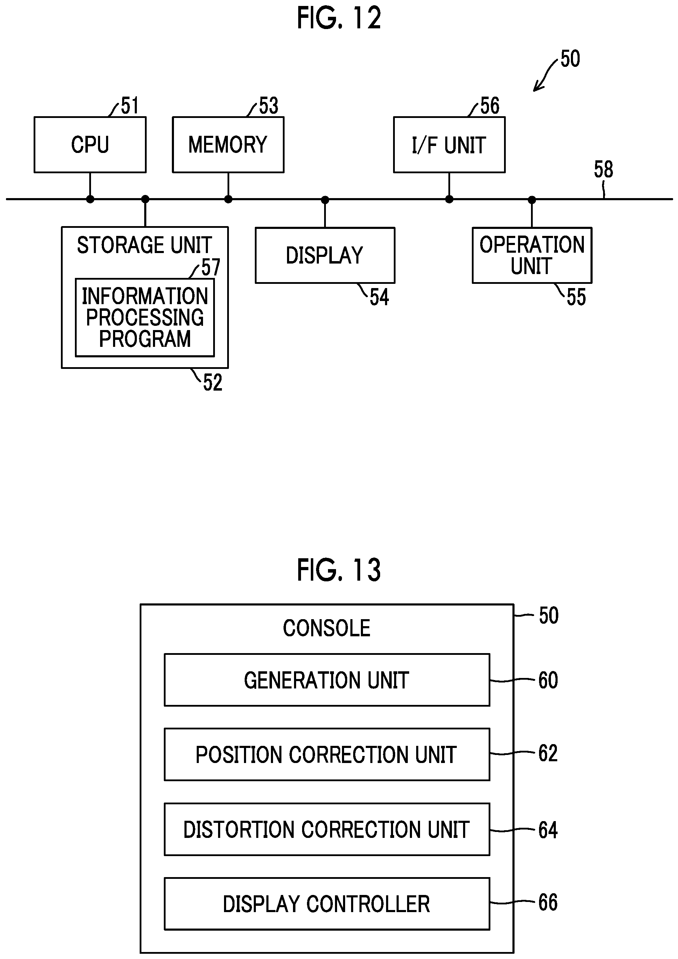

A description of an example of a hardware configuration of the console 50 will be made with reference to Fig. 12. As shown in Fig. 12, the console 50 includes a CPU 51, a nonvolatile storage unit 52, and a memory 53 as a transitory storage region. In addition, the console 50 includes a display 54, such as a liquid crystal display, an operation unit 55, such as a touch panel, a keyboard, and a mouse, and an I/F unit 56. The I/F unit 56 performs wired or wireless communication with the image generation apparatus 10, the RIS 6, and other external apparatuses. The CPU 51, the storage unit 52, the memory 53, the display 54, the operation unit 55, and the I/F unit 56 are connected to each other via a bus 58, such as a system bus and a control bus, so that various types of information can be exchanged.

The storage unit 52 is realized by, for example, a storage medium, such as an HDD, an SSD, and a flash memory. A information processing program 57 in the console 50 is stored in the storage unit 52. The CPU 51 reads out the information processing program 57 from the storage unit 52 to deploy the information processing program 57 into the memory 53, and executes the deployed information processing program 57. As the console 50, for example, a personal computer, a server computer, a smartphone, a tablet terminal, a wearable terminal, or the like can be applied as appropriate.

In addition, the storage unit 52 stores the image data of the radiation image and the ultrasound image acquired by the image generation apparatus 10, various types of other information, and the like. The image data of the radiation image and the ultrasound image may be stored in association with at least one of the imaging order or the imaging information. The imaging information may be, for example, at least one of examinee information and an imaging item that are included in the imaging order, photographer information indicating a photographer (for example, the user, such as the doctor or the technician) who performs the imaging, or date and time information indicating date and time when the imaging is performed.

A description of an example of a functional configuration of the console 50 will be made with reference to Fig. 13. As shown in Fig. 13, the console 50 includes a generation unit 60, a position correction unit 62, a distortion correction unit 64, and a display controller 66. In a case in which the CPU 51 executes the information processing program 57, the CPU 51 functions as the generation unit 60, the position correction unit 62, the distortion correction unit 64, and the display controller 66.

The generation unit 60 according to the present embodiment generates the radiation image and the ultrasound image in a state in which the compression member 40 is used. In addition, the position correction unit 62 according to the present embodiment performs position correction of the ultrasound image and the radiation image by using the first image 70A, which is the image of the first marker 70 included in the ultrasound image, and the second image 72A, which is the image of the second marker 72 included in the radiation image. Then, the display controller 66 according to the present embodiment displays the ultrasound image and the radiation image subjected to the position correction by the position correction unit 62 in a state of being superimposed on each other. As described above, in the present embodiment, the display controller 66 displays the ultrasound image and the radiation image, which are subjected to the position correction by the position correction unit 62, in a state of being superimposed on each other, but the present disclosure is not limited to this. For example, a form may be adopted in which the display controller 66 displays the ultrasound image and the radiation image, which are subjected to the position correction, side by side.

In the present embodiment, the position correction of the ultrasound image and the radiation image by the position correction unit 62 is performed as described below.

First, it is specified which of all the straight lines indicated by the first marker 70 is a straight line indicated by the first image 70A imaged in the ultrasound image, from the position of the ultrasound probe 30 in a case of obtaining the ultrasound image.

Then, at least one of the ultrasound image or the radiation image (in the present embodiment, only the ultrasound image) is moved so that the center of the corresponding second image 72A is located on an extension line of the specified straight line.

On the other hand, in a case in which the display controller 66 performs the display, the distortion correction unit 64 according to the present embodiment performs the distortion correction on at least one of the ultrasound image or the radiation image (in the embodiment, both the ultrasound image and the radiation image).

That is, the compression part 42 of the compression member 40 is strained according to the compression force in a case in which the breast is compressed. Then, in the present embodiment, the distortion correction unit 64 performs the distortion correction on both the ultrasound image and the radiation image.

In the present embodiment, the distortion correction for the ultrasound image and the radiation image is performed by converting the ultrasound image and the radiation image, which are subjected to the position correction, such that a grid composed of the first image 70A imaged in the ultrasound image forms a square in a matrix.

As described above, the distortion correction unit 64 according to the present embodiment performs the distortion correction after the position correction by the position correction unit 62 is performed, but the present disclosure is not limited to this. For example, a form may be adopted in which the distortion correction of each of the ultrasound image and the radiation image by the distortion correction unit 64 is individually performed before the position correction by the position correction unit 62 is performed.

Further, the generation unit 60 according to the present embodiment generates a final ultrasound image by combining the ultrasound images obtained in two stages of pre-scanning in which the position of the first marker 70 provided in the compression member 40 is used as a focal length and main-scanning in which the breast put into the compressed state by the compression member 40 is used as a focal length.

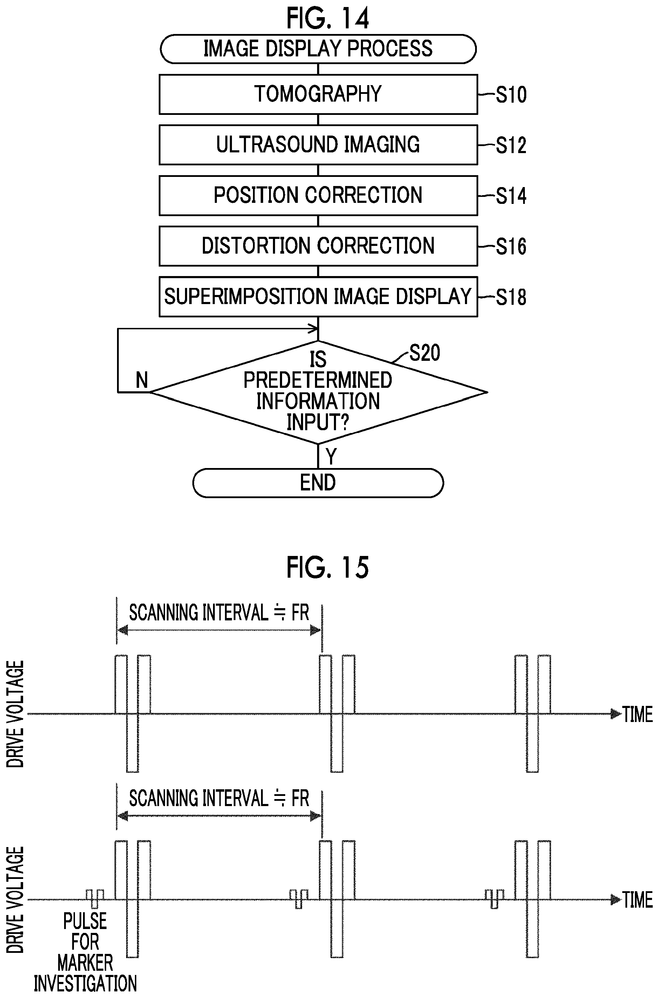

Next, a description of an action of the console 50 according to the present embodiment will be made with reference to Fig. 14. In the console 50, the CPU 51 executes the information processing program 57 to execute an image display process shown in Fig. 14. The image display process is executed, for example, in a case in which the user gives an instruction to start the execution via the operation unit 55. It should be noted that, here, in order to avoid complications, a case is described in which the breast of the examinee is positioned on the imaging surface 16A of the imaging table 16 by the user, such as the doctor or the technician, and is put into the compressed state by the compression member 40.

In step S10, the CPU 51 controls the image generation apparatus 10 to capture the radiation image.

In step S12, the CPU 51 controls the image generation apparatus 10 to capture the ultrasound image.

In this case, the CPU 51 may generate the ultrasound image in one scanning, as shown in the upper figure of Fig. 15 as an example. It should be noted that the upper figure of Fig. 15 is a waveform diagram showing an example of a drive pulse for the ultrasound transducer of the ultrasound probe 30 in a case in which the ultrasound image is generated by one scanning.

However, in this case, since the first marker 70 is located at a position relatively near the ultrasound probe 30, the brightness (reflection of ultrasound waves) in the ultrasound image is increased, and there is a possibility that adverse influence on the imaging and the visibility of the tissue at a deeper portion.

Then, in the present embodiment, as shown in the lower figure of Fig. 15 as an example, the pre-scanning (portion of a pulse for marker investigation shown in the lower figure of Fig. 15) of scanning only the first marker 70 is incorporated in a sequence of generating the ultrasound image. It should be noted that the lower figure of Fig. 15 is a waveform diagram showing an example of a drive pulse for the ultrasound transducer of the ultrasound probe 30 in a case in which the ultrasound image is generated by including the pre-scanning.

In the present embodiment, first, the CPU 51 performs, as the pre-scanning for the first marker 70, beam forming in which only the depth in the vicinity of the compression part 42 is used as the focal length. In addition, the CPU 51 performs the pre-scanning by relatively lowering a drive voltage of the ultrasound probe 30 and relatively reducing a pulse width of the drive pulse.

With this the pre-scanning, halation can be prevented by decreasing the drive voltage, and high resolution can be achieved by reducing the pulse width. As a result, the ultrasound image including the first image 70A can be obtained with high accuracy.

Next, the CPU 51 generates the ultrasound image including the breast image 2A by performing the beam forming at a position deeper than the compression part 42 and using the position of the breast as the focal length, as the main-scanning for the breast.

Then, the CPU 51 generates the final ultrasound image by combining the ultrasound image obtained in the pre-scanning with the ultrasound wave obtained in the main-scanning.

In step S14, the CPU 51 performs the registration between the generated radiation image and ultrasound image, as described above.

It should be noted that, in a case in which the registration is performed, the CPU 51 adjusts the position of the second image 72A in the generated radiation image in accordance with the thickness of the breast as a target.

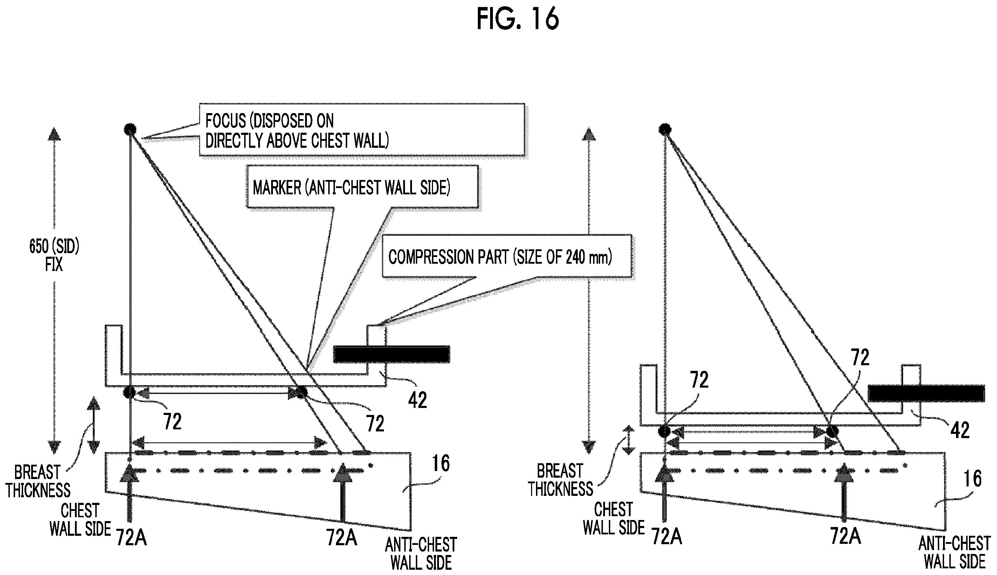

That is, as shown in Fig. 16 as an example, the position of the second image 72A in the generated radiation image is changed in accordance with the thickness of the breast as the target. It should be noted that the left figure of Fig. 16 is a side view showing states of the compression part 42 and the imaging table 16 in a case in which the breast is relatively thick, and the right figure is a side view showing states of the compression part 42 and the imaging table 16 in a case where the breast is thinner than the breast shown in the left figure.

In the example shown in Fig. 16, in a case in which a distance between the second marker 72 provided on the chest wall side on the contact surface 43B of the compression part 42 and the second marker 72 provided on the anti-chest wall side is 200 mm, the distance in the radiation image is 236.4 mm (= 200 × 650/550) in a case in which the thickness of the breast is 100 mm, and the distance in the radiation image is 216.7 mm (= 200 × 650/600) in a case in which the thickness of the breast is 50 mm.

As described above, since the position of the second image 72A in the radiation image is changed in accordance with the thickness of the breast as the target, the CPU 51 adjusts the position of the second image 72A in accordance with the thickness of the breast.

In step S16, the CPU 51 performs the distortion correction of the radiation image and the ultrasound image, which are subjected to the position correction, as described above.

In step S18, the CPU 51 controls the display 54 to display the radiation image and the ultrasound image, which are subjected to the processes described above, in a state of being superimposed on each other, and in step S20, the CPU 51 waits until predetermined information is input. Hereinafter, the image displayed in a state of being superimposed on each other will be referred to as a "superimposition image".

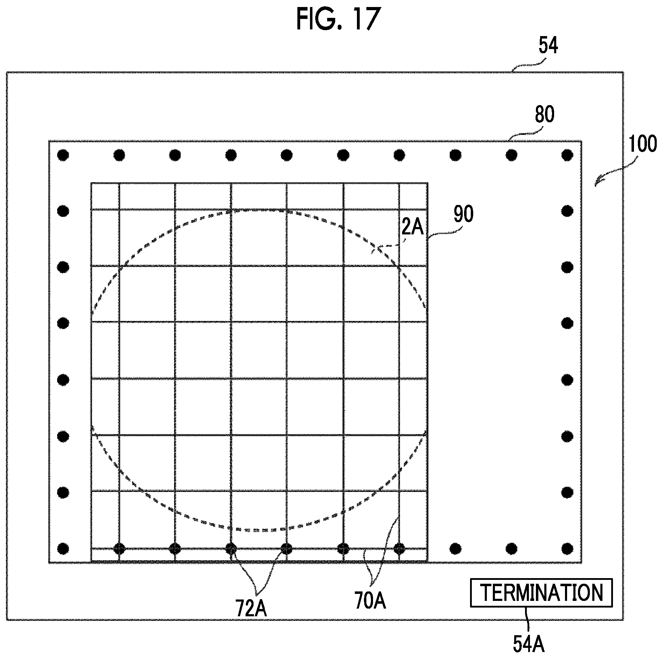

Fig. 17 shows an example of a superimposition image 100 according to the present embodiment. As shown in Fig. 17, in the superimposition image 100, the generated radiation image 80 and ultrasound image 90 are displayed in a state of being superimposed on each other. Therefore, the user can more easily understand the presence or absence of a lesion or the state of the lesion in the breast image 2A by referring to the superimposition image 100.

After referring to the superimposition image 100, the user uses the operation unit 55 to designate a termination button 54A displayed on the screen on which the superimposition image 100 is displayed. In accordance with this designation, an affirmative determination is made in step S20, and the image display process is terminated.

As described above, the compression member 40 according to the present embodiment comprises the first marker 70 that is formed of the first material, which is visible in the ultrasound image and is not visible in the radiation image, and of which the position in the compression member 40 is discriminative, and the second marker 72 that is provided at the position, which does not affect the image interpretation of the radiation image, and is formed of the second material which is visible in the radiation image. Therefore, by performing the registration between the ultrasound image and the radiation image by using the first image 70A, which is the image of the first marker 70, and the second image 72A, which is the image of the second marker 72, it is possible to perform the registration between the radiation image and the ultrasound image with higher accuracy than in the related art.

With the compression member 40 according to the present embodiment, the first material is beryllium. Accordingly, the first image 70A can be detected more accurately as compared to a case in which the first material is a material other than beryllium, and as a result, the registration between the radiation image and the ultrasound image can be performed with higher accuracy.

With the compression member 40 according to the present embodiment, the first marker 70 is disposed on the same axis as the second marker 72. Therefore, it is possible to more easily perform the registration between the radiation image and the ultrasound image as compared to a case in which the first marker 70 is disposed irrespective of the second marker 72.

In addition, with the compression member 40 according to the present embodiment, the first marker 70 is provided on the contact surface 43B which is the surface opposite to the surface with which the ultrasound probe 30 used to capture the ultrasound image is in contact. Therefore, the first image 70A in the ultrasound image can be more easily visible as compared to a case in which the first marker 70 is provided on the upper surface 43A that is a surface with which the ultrasound probe 30 is in contact, and as a result, it is possible to perform the registration between the radiation image and the ultrasound image with higher accuracy.

With the image generation system 1 according to the present embodiment, the compression member 40 described above is provided, and the radiation image and the ultrasound image are generated in a state in which the compression member 40 is used. Therefore, similarly to the compression member 40, it is possible to perform the registration between the radiation image and the ultrasound image with higher accuracy than in the related art.

In addition, with the image generation system 1 according to the present embodiment, the position correction of the ultrasound image and the radiation image is performed by using the first image 70A and the second image 72A, and the ultrasound image and the radiation image, which are subjected to the position correction, are displayed in a state of being superimposed on each other. Therefore, by referring to the displayed image, it is possible to perform the image interpretation by taking both the advantage of the ultrasound image and the advantage of the radiation image.

Further, with the image generation system 1 according to the present embodiment, the final ultrasound image is generated by combining the ultrasound images obtained in the two stages of the pre-scanning in which the position of the first marker 70 provided in the compression member 40 is used as the focal length and the main-scanning in which the breast put into the compressed state by the compression member 40 is used as the focal length. Therefore, the influence of the first image 70A on the ultrasound image can be reduced as compared to a case in which the ultrasound image is generated in one scanning.

It should be noted that, in the embodiment described above, a case is described in which the CPU 51 provided in the console 50 is applied as the processor according to the technique of the present disclosure, but the present disclosure is not limited to this. For example, a form may be adopted in which a CPU of the controller 20 provided in the image generation apparatus 10 is applied as the processor according to the technique of the present disclosure.

In addition, in the embodiment described above, a case is described in which metal that is visible in the radiation image is applied as the material of the second marker 72, but the present disclosure is not limited to this. For example, a form may be adopted in which a colored material that is visible in an image captured by a visible light camera is applied as the material of the second marker 72. In this form, the position of the second marker 72 is specified from the image captured by the visible light camera.

That is, there is a case in which a lesion is generated on the chest wall side in the radiation image, and it is preferable to avoid the influence of the second marker 72 on the radiation image on the chest wall side. In a case of a relatively large breast, there is a possibility that the second marker 72 also affects the radiation image on the opposite side to the chest wall side or in the left-right direction. On the other hand, it is possible to avoid the influence on the radiation image by making the second marker 72 of a material that does not affect the radiation image.

In addition, in this form, a form is preferable in which the first marker 70 is shared as the second marker 72 by performing the coloring on the first marker 70, because the number of markers can be reduced.

In the embodiment described above, a case is described in which the first marker 70 and the second marker 72 are directly provided on the compression part 42 of the compression member 40, but the present disclosure is not limited to this. For example, a form may be adopted in which a sheet made of paper or the like in which the first marker 70 and the second marker 72 are provided is prepared, and the sheet is placed on the compression part 42 only in a case in which the ultrasound image and the radiation image are generated. Since the sheet in this case has adhesiveness, the sheet can be easily attached to the contact surface 43B of the compression part 42.

In addition, although not described in the embodiment described above, a form may be adopted in which the ultrasound image is generated using a so-called space compound method of emitting beams for capturing the ultrasound image from a plurality of directions toward a depth direction to combine the beams in a case in which the ultrasound image is captured. Accordingly, it is possible to give a filtering effect of reducing the noise to the ultrasound image.

In the embodiment described above, a case is described in which the ultrasound probe 30 is automatically moved in a case of generating the ultrasound image, but the present disclosure is not limited to this. For example, a form may be adopted in which a known probe in the related art, such as a one-dimensional probe or a two-dimensional probe, is applied instead of the ultrasound probe 30, and a person may manually move the ultrasound probe to generate the ultrasound image.

It should be noted that, in the embodiment described above, for example, as hardware structures of processing units that execute various types of processes, such as the generation unit 60, the position correction unit 62, the distortion correction unit 64, and the display controller 66, various processors shown below can be used. As described above, in addition to the CPU that is a general-purpose processor that executes software (program) to function as various processing units, the various processors include a programmable logic device (PLD) that is a processor of which a circuit configuration can be changed after manufacture, such as a field programmable gate array (FPGA), and a dedicated electric circuit that is a processor having a circuit configuration that is designed for exclusive use in order to execute a specific process, such as an application specific integrated circuit (ASIC).

One processing unit may be configured by using one of the various processors or may be configured by using a combination of two or more processors of the same type or different types (for example, a combination of a plurality of FPGAs or a combination of a CPU and an FPGA). Moreover, a plurality of processing units may be configured of one processor.

A first example of the configuration in which the plurality of processing units are configured by using one processor is a form in which one processor is configured by using a combination of one or more CPUs and the software and this processor functions as the plurality of processing units, as represented by computers, such as a client and a server. A second example is a form of using a processor that realizes the function of the entire system including the plurality of processing units by one integrated circuit (IC) chip, as represented by a system on chip (SoC) or the like. In this way, as the hardware structure, the various processing units are configured by using one or more of the various processors described above.

Further, the hardware structure of these various processors is, more specifically, an electric circuit (circuitry) in which circuit elements such as semiconductor elements are combined.

In addition, in the embodiment described above, the aspect is described in which the information processing program 57 is stored (installed) in the storage unit 52 of the console 50 in advance, but the present disclosure is not limited to this. The information processing program 57 may be provided in a form of being recorded in a recording medium, such as a compact disc read only memory (CD-ROM), a digital versatile disc read only memory (DVD-ROM), and a universal serial bus (USB) memory. In addition, a form may be adopted in which the information processing program 57 is downloaded from an external apparatus via the network.

## Claims

1. A compression member (40, 40S) that puts a breast disposed between a radiation source (17R) and a radiation detector (28) into a compressed state, the compression member (40, 40S) comprising:
a first marker (70) that is formed of a first material, which is visible in an ultrasound image (90) and is not visible in a radiation image (80), and of which a position in the compression member (40, 40S) is discriminative; and
a second marker (72) that is provided at a position, which does not affect an image interpretation of the radiation image (80), and is formed of a second material which is visible in the radiation image (80);
wherein the first marker (70) is provided on a surface (43B) opposite to a surface with which an ultrasound probe (30) used to capture the ultrasound image (90) is in contact.

2. The compression member (40, 40S) according to claim 1,
wherein the first material is beryllium.

3. The compression member (40, 40S) according to claim 1 or 2,
wherein the first marker (70) is disposed on the same axis as the second marker (72).

4. The compression member (40, 40S) according to any one of claims 1 to 3,
wherein the first marker (70) is disposed in a patterned manner associated with position information in the compression member (40, 40S).

5. An image generation apparatus (10) comprising:
a radiation source (17R);
a radiation detector (28);
an imaging table (16) disposed between the radiation source (17R) and the radiation detector (28);
a compression member (40, 40S) according to any of one of claims 1 to 4;
an ultrasound probe (30); a controller (20) and
at least one processor (51),
wherein the image generation apparatus (10) generates a radiation image (80) and an ultrasound image (90) in a state in which the compression member (40, 40S) is used.

6. The image generation apparatus (10) according to claim 5,
wherein the processor (51)
performs position correction of the ultrasound image (90) and the radiation image (80) by using a first image (70A), which is an image of the first marker (70) included in the ultrasound image (90), and a second image (72A), which is an image of the second marker (72) included in the radiation image (80), and
displays the ultrasound image (90) and the radiation image (80), which are subjected to the position correction, side by side or displays the ultrasound image (90) and the radiation image (80), which are subjected to the position correction, in a state of being superimposed on each other.

7. The image generation apparatus (10) according to claim 6,
wherein the processor (51) performs distortion correction on at least one of the ultrasound image (90) or the radiation image (80) in a case in which the display is performed.

8. The image generation apparatus (10) according to claim 6 or 7,
wherein the processor (51) generates the ultrasound image (90) by combining ultrasound images obtained in two stages of pre-scanning in which the position of the first marker (70) provided in the compression member (40, 40S) is used as a focal length and main-scanning in which the breast put into the compressed state by the compression member (40, 40S) is used as a focal length.

9. The image generation apparatus (10) according to any one of claims 6 to 8,
wherein, in a case in which the ultrasound image (90) is captured, the image generation apparatus (10) emits beams for capturing the ultrasound image (90) from a plurality of directions toward a depth direction to combine the beams.

10. A non-transitory storage medium storing a program executed in an image generation apparatus (10) according to claim 5, the program for causing the controller (20) to execute a process comprising:
generating a radiation image (80) and an ultrasound image (90) in a state in which the compression member (40, 40S) is used.

## Patentansprüche

1. Kompressionselement (40, 40S), das eine Brust, die zwischen einer Strahlungsquelle (17R) und einem Strahlungsdetektor (28) angeordnet ist, in einen komprimierten Zustand versetzt, wobei das Kompressionselement (40, 40S) umfasst:
eine erste Markierung (70), die aus einem ersten Material gebildet ist, das in einem Ultraschallbild (90) sichtbar ist und in einem Strahlungsbild (80) nicht sichtbar ist, und von der eine Position bei dem Kompressionselement (40, 40S) diskriminierend ist; und
eine zweite Markierung (72), die an einer Position vorgesehen ist, die eine Bildauswertung des Strahlungsbildes (80) nicht beeinflusst, und aus einem zweiten Material gebildet ist, das in dem Strahlungsbild (80) sichtbar ist,
wobei die erste Markierung (70) auf einer Fläche (43B) gegenüber einer Fläche vorgesehen ist, mit der eine Ultraschallsonde (30), die zum Aufnehmen des Ultraschallbildes (90) verwendet wird, in Kontakt steht.

2. Kompressionselement (40, 40S) nach Anspruch 1,
wobei das erste Material Beryllium ist.

3. Kompressionselement (40, 40S) nach Anspruch 1 oder 2,
wobei die erste Markierung (70) auf derselben Achse wie die zweite Markierung (72) angeordnet ist.

4. Kompressionselement (40, 40S) nach einem der Ansprüche 1 bis 3,
wobei die erste Markierung (70) auf eine gemusterte Weise, die mit Positionsinformationen bei dem Kompressionselement (40, 40S) verknüpft ist, angeordnet ist.

5. Bilderzeugungsvorrichtung (10), umfassend:
eine Strahlungsquelle (17R);
einen Strahlungsdetektor (28);
einen Bildgebungstisch (16), der zwischen der Strahlungsquelle (17R) und dem Strahlungsdetektor (28) angeordnet ist;
ein Kompressionselement (40, 40S) nach einem der Ansprüche 1 bis 4;
eine Ultraschallsonde (30);
eine Steuerung (20); und
mindestens einen Prozessor (51),
wobei die Bilderzeugungsvorrichtung (10) ein Strahlungsbild (80) und ein Ultraschallbild (90) in einem Zustand erzeugt, in dem das Kompressionselement (40, 40S) verwendet wird.

6. Bilderzeugungsvorrichtung (10) nach Anspruch 5,
wobei der Prozessor (51)
Positionskorrektur des Ultraschallbildes (90) und des Strahlungsbildes (80) durch Verwenden eines ersten Bildes (70A), das ein Bild der ersten Markierung (70) ist, das in dem Ultraschallbild (90) enthalten ist, und eines zweiten Bildes (72A), das ein Bild der zweiten Markierung (72) ist, das in dem Strahlungsbild (80) enthalten ist, durchführt, und
das Ultraschallbild (90) und das Strahlungsbild (80), die der Positionskorrektur unterzogen worden ist, Seite an Seite anzeigt oder das Ultraschallbild (90) und das Strahlungsbild (80), die der Positionskorrektur unterzogen worden sind, in einem Zustand anzeigt, in dem sie einander überlagert sind.

7. Bilderzeugungsvorrichtung (10) nach Anspruch 6,
wobei der Prozessor (51) Verzeichnungskorrektur an mindestens einem von dem Ultraschallbild (90) und dem Strahlungsbild (80) in einem Fall, in dem die Anzeige durchgeführt wird, durchführt.

8. Bilderzeugungsvorrichtung (10) nach Anspruch 6 oder 7,
wobei der Prozessor (51) das Ultraschallbild (90) durch Kombinieren von Ultraschallbildern erzeugt, die in zwei Phasen von Vorscannen, bei dem die Position der ersten Markierung (70), die bei dem Kompressionselement (40, 40S) vorgesehen ist, als eine Brennweite verwendet wird, und Hauptscannen, bei dem die durch das Kompressionselement (40, 40S) in den komprimierten Zustand versetzte Brust als eine Brennweite verwendet wird, erhalten werden.

9. Bilderzeugungsvorrichtung (10) nach einem der Ansprüche 6 bis 8,
wobei in einem Fall, in dem das Ultraschallbild (90) aufgenommen wird, die Bilderzeugungsvorrichtung (10) Strahlen zum Aufnehmen des Ultraschallbildes (90) aus mehreren Richtungen in eine Tiefenrichtung emittiert, um die Strahlen zu kombinieren.

10. Nicht flüchtiges Speichermedium, das ein Programm speichert, das in einer Bilderzeugungsvorrichtung (10) nach Anspruch 5 ausgeführt wird, wobei das Programm die Steuerung (20) veranlasst, einen Prozess auszuführen, der umfasst: Erzeugen eines Strahlungsbildes (80) und eines Ultraschallbildes (90) in einem Zustand, in dem das Kompressionselement (40, 40S) verwendet wird.

## Revendications

1. Élément de compression (40, 40S) qui met un sein disposé entre une source de rayonnement (17R) et un détecteur de rayonnement (28) dans un état comprimé, l'élément de compression (40, 40S) comprenant :
un premier marqueur (70) qui est constitué d'un premier matériau, qui est visible sur une image ultrasonore (90) et n'est pas visible sur une image de rayonnement (80), et dont une position dans l'élément de compression (40, 40S) est identifiable ; et
un deuxième marqueur (72) qui est disposé à une position qui n'affecte pas une interprétation d'image de l'image de rayonnement (80), et est constitué d'un deuxième matériau qui est visible sur l'image de rayonnement (80),
dans lequel le premier marqueur (70) est disposé sur une surface (43B) opposée à une surface avec laquelle une sonde ultrasonore (30) utilisée pour acquérir l'image ultrasonore (90) est en contact.

2. Élément de compression (40, 40S) selon la revendication 1,
dans lequel le premier matériau est du béryllium.

3. Élément de compression (40, 40S) selon la revendication 1 ou la revendication 2, dans lequel le premier marqueur (70) est disposé sur le même axe que le deuxième marqueur (72).

4. Élément de compression (40, 40S) selon l'une quelconque des revendications 1 à 3, dans lequel le premier marqueur (70) est disposé de manière structurée associée à des informations de position dans l'élément de compression (40, 40S).

5. Appareil de génération d'images (10) comprenant :
une source de rayonnement (17R) ;
un détecteur de rayonnement (28) ;
une table d'imagerie (16) disposée entre la source de rayonnement (17R) et le détecteur de rayonnement (28) ;
un élément de compression (40, 40S) selon l'une quelconque des revendications 1 à 4 ;
une sonde ultrasonore (30) ;
un contrôleur (20) ; et
au moins un processeur (51),
dans lequel l'appareil de génération d'images (10) génère une image de rayonnement (80) et une image ultrasonore (90) dans un état où l'élément de compression (40, 40S) est utilisé.

6. Appareil de génération d'images (10) selon la revendication 5,
dans lequel le processeur (51)
effectue une correction de position de l'image ultrasonore (90) et de l'image de rayonnement (80) en utilisant une première image (70A), qui est une image du premier marqueur (70) inclus dans l'image ultrasonore (90), et une deuxième image (72A), qui est une image du deuxième marqueur (72) inclus dans l'image de rayonnement (80), et affiche l'image ultrasonore (90) et l'image de rayonnement (80), qui sont soumises à la correction de position, côte à côte ou affiche l'image ultrasonore (90) et l'image de rayonnement (80), qui sont soumises à la correction de position, dans un état où elles sont superposées l'une sur l'autre.

7. Appareil de génération d'images (10) selon la revendication 6,
dans lequel le processeur (51) effectue une correction de distorsion sur au moins l'une de l'image ultrasonore (90) ou de l'image de rayonnement (80) dans un cas où l'affichage est effectué.

8. Appareil de génération d'images (10) selon la revendication 6 ou la revendication 7, dans lequel le processeur (51) génère l'image ultrasonore (90) en combinant des images ultrasonores obtenues lors de deux étapes, à savoir un pré-balayage dans lequel la position du premier marqueur (70) disposé dans l'élément de compression (40, 40S) est utilisée comme distance focale et un balayage principal dans lequel le sein mis dans l'état comprimé par l'élément de compression (40, 40S) est utilisé comme distance focale.

9. Appareil de génération d'images (10) selon l'une quelconque des revendications 6 à 8, dans lequel, dans un cas où l'image ultrasonore (90) est acquise, l'appareil de génération d'images (10) émet des faisceaux pour acquérir l'image ultrasonore (90) à partir d'une pluralité de directions vers une direction de profondeur pour combiner les faisceaux.

10. Support de stockage non transitoire stockant un programme exécuté dans un appareil de génération d'images (10) selon la revendication 5, le programme destiné à amener le contrôleur (20) à exécuter un processus comprenant :
générer une image de rayonnement (80) et une image ultrasonore (90) dans un état où l'élément de compression (40, 40S) est utilisé.
